(19) Europäisches Patentamt
European Patent Office
Office européen des brevets

(11) **EP 2 378 984 B1**

## (12) FASCICULE DE BREVET EUROPEEN

(45) Date de publication et mention
de la délivrance du brevet:
**23.08.2017 Bulletin 2017/34**

(21) Numéro de dépôt: **09760938.2**

(22) Date de dépôt: **16.11.2009**

(51) Int Cl.:
***A61B 17/08*** *(2006.01)*    ***A61B 17/122*** *(2006.01)*
***A61B 17/00*** *(2006.01)*    ***A61B 17/064*** *(2006.01)*

(86) Numéro de dépôt international:
**PCT/FR2009/001310**

(87) Numéro de publication internationale:
**WO 2010/055232 (20.05.2010 Gazette 2010/20)**

(54) **CLIPS D'ANASTOMOSE VASCULAIRE**

KLAMMERN FÜR GEFÄSSANASTOMOSEN

CLIPS FOR VASCULAR ANASTOMOSIS

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**

(30) Priorité: **17.11.2008 FR 0806418**

(43) Date de publication de la demande:
**26.10.2011 Bulletin 2011/43**

(73) Titulaire: **Sarradon, Pierre**
**83000 Toulon (FR)**

(72) Inventeur: **Sarradon, Pierre**
**83000 Toulon (FR)**

(74) Mandataire: **Alatis**
**Cabinet d'Avocats**
**22 rue Drouot**
**75009 Paris (FR)**

(56) Documents cités:
**EP-A- 1 462 061    WO-A-03/099139**
**US-A1- 2008 173 693**

## Description

### DOMAINE TECHNIQUE DE L'INVENTION

La présente invention concerne le domaine de la chirurgie et plus particulièrement le domaine de la chirurgie vasculaire et, de manière non limitative, la microchirurgie vasculaire sur l'animal ou sur l'homme.

### ETAT DE LA TECHNIQUE

Dans de nombreuses procédures chirurgicales, il est nécessaire d'aboucher des vaisseaux sanguins: la procédure de réunion de conduits sanguins, tels que des vaisseaux, des prothèses ou des greffons, mis en communication l'un à l'autre est connue sous le nom d'anastomose vasculaire.

Jusqu'à une période récente, l'anastomose vasculaire s'effectuait uniquement par couture à l'aiguille et au fil. Cependant, cette procédure à l'aide de fils et d'aiguille est longue et fastidieuse pour le chirurgien. En outre, cette technique, qui requiert la perforation de la paroi des vaisseaux aux points de pénétration de l'aiguille, n'est pas totalement exempte de risque ou d'effets néfastes.

On a donc vu se développer ces dernières années en chirurgie artérielle l'utilisation de clips vasculaires de suture, de pinces et de micro-pinces chirurgicales, analogues à celles présentées, par exemple, dans les demandes de brevets EP 1 712 186, FR 2 557 448 et FR 2 588 467. Ces clips, en forme générale de U, se composent principalement de deux branches pouvant se resserrer autour de tissus sanguins réunis, afin de les maintenir ensemble. Ce type de clips vasculaires permet bien d'éviter la formation de caillots dans les vaisseaux puisqu'ils ne traversent pas les tissus. Par ailleurs, la mise en place des clips peut être simplifiée par l'utilisation d'une pince distributrice telle que décrite par exemple dans le document EP 0 656 191, de sorte que l'application de clip permet globalement un gain de temps au chirurgien. Cependant, les clips d'anastomose vasculaire ne sont utilisés que sur de petits vaisseaux car dans le cas de vaisseaux de diamètre important et notamment sur les artères principales, la pression est trop importante, si bien que les clips connus ne permettent pas de maintenir, sur le long terme, les vaisseaux réunis. On a d'ailleurs constaté cliniquement des cas de glissement des clips qui finissent par se libérer complètement, avec un risque concomitant d'ouverture de la jonction et d'hémorragie.

On connaît également depuis quelques années des systèmes d'agrafes, utilisées lors d'anastomoses intestinales par exemple. Ces agrafes pénétrantes résistent à une plus grande pression. Cependant, à supposer qu'on les applique sur des vaisseaux sanguins, elles requerraient la perforation de la paroi des vaisseaux et le maintien d'une partie métallique à l'intérieur du vaisseau, ce qui pourrait provoquer la formation d'agrégations plaquettaires susceptibles d'entraîner un épaississement local des parois des vaisseaux et un rétrécissement de la lumière intérieure des vaisseaux, voire la formation de caillots. Par ailleurs, la perforation engendrerait un risque de déchirure des vaisseaux.

Le document EP 1 462 061 décrit un clip d'hémostase, c'est-à-dire destiné à interrompre un petit vaisseau en remplaçant une ligature. Ce clip est destiné à se déformer élastiquement et non pas plastiquement ou par effet de mémoire de forme.

Le document US2008/173693 décrit un clip destiné à refermer une incision pratiquée dans un vaisseau après une intervention sur ce dernier. Les branches du clip se croisent après déformation. Le clip est pourvu de butées destinées à limiter la pénétration des extrémités du clip dans le vaisseau. Ces butées évitent que les extrémités du clip débouchent dans la lumière du vaisseau.

Le document 003/099139 décrit quant à lui un clip dont les extrémités sont pourvues de crochets qui, dans une position d'utilisation, sont déformés plastiquement pour se refermer sur des reliefs correspondants de la branche opposée au clip. Les crochets ne sont jamais pris dans les chairs du patient. Le clip présente une base dimensionnée de manière à ne pas se déformer dans des conditions normales d'utilisation.

### OBJET DE L'INVENTION

La présente invention vise à pallier les inconvénients de l'état de la technique en proposant un clip chirurgical permettant une anastomose vasculaire chez l'homme ou l'animal, et en particulier une anastomose vasculaire sur des conduits sanguins de tout diamètre et notamment de diamètre important, notamment les artères principales, qui soit durable, rapide, et qui limite autant que possible les problèmes d'agrégation plaquettaire, d'épaississement de vaisseaux, de réduction de la section du conduit sanguin ou de formation de caillots dans les conduits sanguins. On entend dans la présente demande par conduit sanguin aussi bien les vaisseaux sanguins que les greffons et les prothèses vasculaires.

Par ailleurs, le dispositif selon l'invention ne permet pas seulement l'anastomose vasculaire mais il permet également la suture de conduits sanguins.

Pour cela, la présente invention propose d'utiliser un clip chirurgical à déformation plastique ou à mémoire de forme, comportant une base de laquelle font saillie au moins deux branches présentant chacune un crochet pointant vers la base. La base forme un V déformable à son sommet, de sorte que le clip comporte trois zones de déformation privilégiée, à savoir le sommet et les zones de raccordement entre la base et les branches.

Ces crochets permettent un ancrage du clip dans les parois vasculaires à suturer, sans toutefois les perforer, afin d'assurer le maintien du clip, et ce, même en présence de fortes pressions artérielles comme c'est le cas dans les vaisseaux de grand diamètre. La déformation du clip, qu'elle soit obtenue plastiquement ou par effet mémoire d'un alliage à mémoire de forme, permet quant à elle un maintien satisfaisant des tissus. Un tel maintien ne pourrait pas par exemple être obtenu par déformation élastique du clip et des tissus.

Dans un mode particulier de réalisation de la présente invention, la base est déformable plastiquement ou par effet mémoire pour rapprocher les branches du clip, de façon à ce que les crochets se plantent dans les parois vasculaires à réunir, sans toutefois les perforer.

Préférentiellement, chaque branche forme avec son crochet un coin rentrant d'angle au sommet compris entre 30° et 80°. Ces angles permettent une bonne pénétration et un bon ancrage des crochets dans les chairs.

Avantageusement, les extrémités libres des branches peuvent être arrondies afin d'éviter l'endommagement physiologique des tissus.

Selon un mode de réalisation de l'invention, les branches du clip avant déformation sont parallèles.

Selon un mode de réalisation, le clip présente deux faces planes parallèles couvrant chacune la base et les deux branches, les deux faces planes étant situées à une distance relative P comprise entre 0,5 et 3 mm et, de préférence, inférieure 1,5 mm. Il est ainsi possible de stocker les clips en pile formant des barrettes qui peuvent être insérées dans une pince de distribution.

Le clip a une hauteur H, mesurée parallèlement aux faces planes parallèles, qui est préférentiellement comprise entre 1 et 5 mm. Chaque crochet comporte une extrémité libre qui se trouve à une distance E de la face interne de la branche à laquelle est relié ce crochet, cette distance E vérifiant avantageusement l'inégalité:

$$0,05*H < E < 0,4*H \text{ et, de préférence, } 0,1*H < E < 0,3*H.$$

Avantageusement, l'épaisseur P du clip est telle que:

$$0,1*H < P < 0,4*H.$$

De plus, le clip selon la présente invention est composé d'un matériau biocompatible, qui peut être, selon un mode de réalisation préféré, du titane, afin d'être toléré par le corps humain.

Alternativement, un alliage à mémoire de forme, notamment un nitinol tel que décrit par exemple dans le document FR2899113.

Avantageusement le clip est réalisé par une pièce d'un seul tenant afin de faciliter sa fabrication.

Le clip est utilisable notamment pour les anastomoses termino-terminales, latéro-terminales, latéro-latérales pour les sutures latérales et pour l'application de patchs.

La présente invention concerne également une méthode d'anastomose vasculaire termino-terminale entre un premier conduit sanguin et un deuxième conduit sanguin, caractérisée en ce qu'elle comporte les étapes suivantes:

- retrousser partiellement une paroi d'extrémité du premier conduit sanguin;

- insérer l'extrémité retroussée du premier conduit dans une paroi d'extrémité du deuxième conduit sanguin pour former une jonction continue entre les deux parois d'extrémités;

- positionner à cheval sur les deux extrémités en un point de la jonction un clip comportant deux branches présentant chacun une extrémité libre arrondie et un crochet sur la face interne de chacune des branches;

- fermer ledit clip par déformation plastique ou par effet mémoire de manière à ce que les crochets pénètrent dans les parois d'extrémité des deux conduits sanguins, sans toutefois les traverser;

- répéter les opérations de positionnement et de fermeture sur l'ensemble de la jonction.

L'invention concerne également une méthode d'anastomose vasculaire termino-latérale entre une paroi d'extrémité d'un premier conduit sanguin et une paroi latérale d'un deuxième conduit sanguin, caractérisée en ce qu'elle comporte les étapes suivantes:

- retrousser partiellement la paroi d'extrémité du premier conduit sanguin;

- pratiquer une incision dans la paroi latérale du deuxième conduit sanguin;

- insérer la paroi d'extrémité retroussée du premier conduit sanguin dans l'incision de la paroi latérale du deuxième conduit sanguin pour former une jonction entre les parois; - positionner à cheval sur la paroi d'extrémité retroussée et un bord de l'incision un clip comportant deux branches présentant chacun une extrémité libre arrondie et un crochet sur la face interne de chacune des branches;

- fermer ledit clip par déformation plastique ou par effet mémoire de manière à ce que les crochets pénètrent dans les parois, sans toutefois les traverser.

- répéter les opérations de positionnement et de fermeture sur l'ensemble de la jonction.

Les conduits sanguins concernés peuvent inclure des vaisseaux, des prothèses ou des greffons.
Selon un mode particulier de réalisation, les clips sont disposés tout autour de la périphérie de la jonction, de proche en proche à un millimètre d'intervalle.

## BREVE DESCRIPTION DES FIGURES

D'autres caractéristiques et avantages de l'invention ressortiront à la lecture de la description qui suit, en référence aux figures annexées, qui illustrent :

- la figure 1, une vue en perspective d'un clip chirurgical selon un premier mode de réalisation de la présente invention dans une position initiale non déformée;

- la figure 2, une vue en coupe du clip de la figure 1, pincé pour suturer bout à bout deux vaisseaux;

- la figure 3, une vue de face d'un fil métallique utilisé pour réaliser un clip chirurgical à mémoire de forme;

- la figure 4, une vue de face d'un clip chirurgical selon un deuxième mode de réalisation de la présente invention dans sa position initiale non déformée.

- la figure 5, une vue de côté du clip chirurgical selon le mode de réalisation de la figure 4, et plus particulièrement une vue de la branche 2.

- la figure 6, une vue de la section du clip selon le mode de réalisation de la figure 4.

## EXEMPLE DE REALISATION

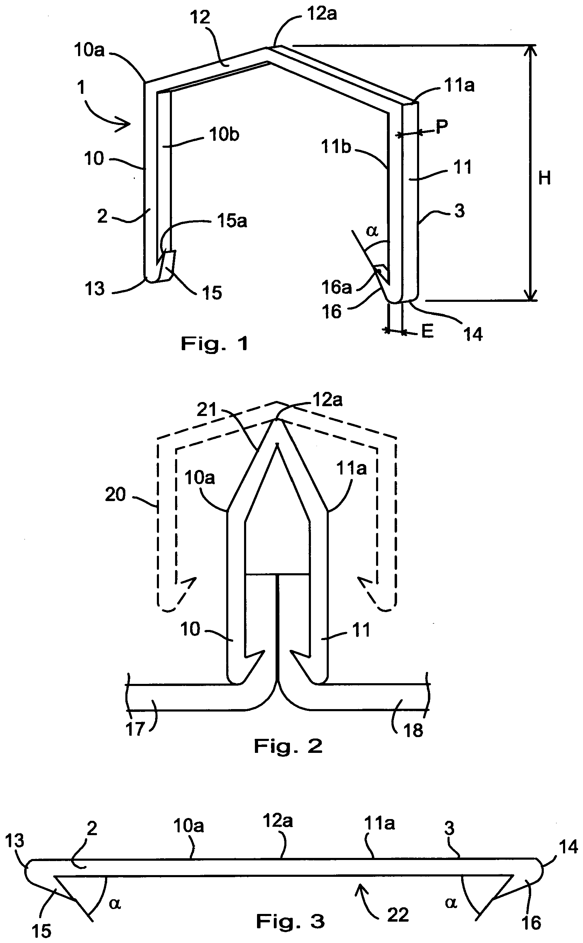

En référence à la figure 1, un clip chirurgical 1 conforme à l'invention est constitué par une pièce d'un seul tenant, en un matériau biocompatible et déformable plastiquement, comme par exemple du titane. Le clip est formé dans une plaque plane, par exemple par découpage laser ou électrochimique, et comporte de ce fait deux faces 2, 3 planes parallèles dont la distance relative détermine l'épaisseur P constante du clip.
Le clip comporte deux branches d'extrémité 10 et 11 parallèles, interconnectées par une base 12 formant un V de sommet 12a. Les extrémités libres 13 et 14 des deux branches sont arrondies afin d'éviter toute déchirure des tissus et tout caillot. Faisant partie intégrante des branches 10, 11, deux crochets 15 ou 16 pointent l'un vers l'autre et vers la base 12. L'extrémité de chacun des crochets 15 et 16 est de préférence en forme d'arête. Les branches font un angle ouvert de sommet 10a, 11 a avec la base 12. De manière remarquable, chaque crochet a une face 15a, 16a tournée vers la face interne 10b, 11 b de la branche correspondante, formant un coin rentrant d'angle rentrant $\alpha$.
Les dimensions du clip varient en fonction de la taille des conduits sanguins auxquels il est destiné. L'épaisseur P du clip est de l'ordre de 0,5 à 1,5 mm. La hauteur H du clip, mesurée entre le sommet 12a et un plan perpendiculaire au plan du clip et joignant les extrémités libres 13, 14 varie ainsi entre 2 et 5 mm. La distance E entre l'extrémité libre du crochet et la face intérieure de la branche correspondante varie entre 0,1 et 0,3 fois H. L'angle aigu $\alpha$ est toujours inférieur à 90° et varie préférentiellement entre 30° et 60° ou 80°.
La figure 2 montre le même clip 20 en pointillés avant déformation plastique. Cette figure montre également en traits pleins ce même clip 21, qui enserre deux tissus 17 et 18 après déformation plastique du clip. Le clip a été disposé à califourchon autour des deux tissus, puis il a été serré autour des tissus par déformation plastique de façon à ce que les crochets 15, 16 se plantent dans les tissus, sans toutefois les traverser. Après déformation plastique, les deux branches 10 et 11 du clip restent sensiblement parallèles. La déformation du clip s'est concentrée sur le sommet 12a de la base dont l'angle s'est rétréci sous la sollicitation de la pince, et sur les sommets 10a, 11 a qui se sont ouverts.

Les crochets, dirigés vers la base du clip, ont pénétré dans les tissus sans les traverser et réalisent un ancrage du clip qui empêche le glissement des tissus hors du clip, et ce, même en présence d'une forte pression sanguine ou en cas de sollicitation mécanique extérieure.

Pour mettre en place le clip dans un cas d'anastomose termino-terminale, on procède de la manière suivante. Dans un premier temps, les extrémités de deux conduits sanguins sont rapprochées à l'aide de moyens appropriés, l'une d'elles étant retroussées, c'est-à-dire rabattues extérieurement. Un clip, tenu dans une pince à clips, est positionné à califourchon au niveau d'un point de contact des tissus de ces conduits sanguins destiné à la jonction entre les conduits sanguins. La pince permet ensuite de serrer le clip autour des tissus des conduits sanguins, de façon à ce que ses branches soient rapprochées parallèlement autour des tissus et à ce que les crochets se plantent dans les tissus, sans les transpercer. Ce serrage a lieu par déformation plastique de la base à l'aide de la pince ou de tout autre moyen permettant le serrage du clip. La déformation des sommets 10a et 11 a est induite par la résistance des tissus. La déformation plastique du clip est permanente. Cette opération est répétée en autant de points que cela est nécessaire afin de réunir les tissus choisis. La déformation de la base permet au clip de maintenir les tissus ensemble, sans les perforer. Par ailleurs, les crochets évitent que les tissus ne glissent dans le clip et ce, même en présence de fortes pressions, comme c'est le cas dans les vaisseaux sanguins de grande taille. De plus ces crochets ne perforent pas les tissus, si bien qu'ils évitent la formation de caillots dans les vaisseaux.

L'opération est réitérée sur toute la périphérie de la jonction entre les deux tissus. Puis une traction est exercée sur les conduits de manière à déplier l'extrémité du conduit sanguin précédemment retroussée et à tester la solidité de la jonction réalisée.

On procède de manière analogue pour des anastomoses latéro-terminales ou latéro-latérales.

Ces clips fournissent donc un moyen rapide de suture des tissus et sont d'une application nettement plus aisée pour le chirurgien que les procédés classiques, comme par exemple la suture au fil et à l'aiguille. Ces clips permettent en outre de pratiquer l'anastomose sur tout type de vaisseaux sanguins, y compris sur les plus gros, en évitant les inconvénients des autres techniques.

Naturellement, de nombreuses variantes sont possibles. La base peut par exemple être conformée en arc de cercle. On peut également envisager d'avoir un clip de section carrée, rectangulaire ou même ronde suivant les procédés de fabrication du clip et les matériaux dans lequel il est réalisé. Par ailleurs, le clip peut avoir plus de deux branches, par exemple 5 branches disposées de manière circulaire autour de la base, ce qui permet par exemple de fermer l'extrémité d'un vaisseau. De plus, on peut envisager de réaliser le clip en tout matériau biocompatible, y compris en polymère, à condition que le matériau choisi soit déformable plastiquement et soit suffisamment ductile pour garder sa forme lorsque le clip est en position serrée.

Selon une autre variante, le clip peut être réalisé en un alliage à mémoire de forme, à effet simple sens à base de nickel, par exemple celui connu sous la marque "Nitinol". Le clip est fabriqué à partir d'un fil métallique linéaire, de section carrée et d'épaisseur comprise entre 0,3 et 1,5 mm semblable à celui représenté sur la figure 3. Ce fil comprend à chacune de ses extrémités un crochet 15 ou 16 formant un angle aigu avant le fil linéaire. Les deux extrémités du fil 13 et 14 sont arrondies pour ne pas blesser les tissus, de sorte que le clip a une forme initiale analogue à celle représentée sur les figures 1 et 2.

Dans un premier temps, le clip est conformé, à partir du fil 22, par déformation mécanique des points 10a, 11 a et 12a, à une température élevée, voisine de 700°C, pour qu'il occupe sa forme initiale 20.

Le clip est ensuite éduqué de façon à mémoriser sa forme de verrouillage 21. Cette mémorisation s'effectue par déformation mécanique et chauffage simultané, à une température voisine de 700°C. Le clip est chauffé particulièrement au niveau de ses sommets 10a, 11 a et 12a, qui sont déformés pour que le clip occupe sa forme finale 21.

On refroidit ensuite brutalement le clip en le plongeant dans un bain froid, à une température comprise entre -26°C environ et + 20°C, afin de stopper l'éducation selon la forme 21. Le clip se trouve alors dans sa position verrouillée 21.

On procède alors à un premier traitement thermique, à une température voisine de 600°C. La cuisson dure moins de 10 minutes. On procède ensuite à un second traitement thermique, à une température voisine de 300°C. Cette étape de second traitement thermique dure 10 minutes environ.

On dépose ensuite sur le clip une couche d'un matériau biocompatible tel que le titane et on ramène le clip dans sa position initiale 20 par déformation mécanique effectuée à une température comprise entre 18 et 25°C.

Ce procédé d'éducation du clip est connu de l'art antérieur et plus largement décrit dans le document FR2899113.

Bien sûr d'autres procédés de fabrication du clip peuvent être utilisés et celui-ci n'est donné qu'à titre d'illustration.

Ainsi, lorsque le clip se trouve à une température comprise entre 20 et 35°C, il reste dans sa conformation initiale 20. Puis, lors de la pose du clip, le chirurgien met en place le clip autour des tissus à rassembler comme dans le mode de réalisation précédent et lorsque le clip se trouve à une température supérieure ou égale à 35°C, il retrouve sa forme de verrouillage 21, dans laquelle il maintient les tissus ensemble comme dans le mode de réalisation précédent.

L'utilisation d'un clip réalisé en un alliage à mémoire de forme permet de serrer le clip toujours de la même manière, ce qui n'est pas toujours le cas lorsque le serrage du clip est mécanique. Ainsi, le serrage du clip et le positionnement des crochets dans les tissus à rapprocher sont plus précis et plus réguliers.

On peut également envisager d'utiliser des alliages à mémoire de forme double sens, qui peuvent mémoriser deux conformations, et en particulier une conformation permettant l'extraction du clip. On peut également envisager de serrer le clip autour des tissus par déformation mécanique comme dans l'exemple précédent, et sous l'effet de la température, le clip maintiendra sa position de verrouillage 21, s'opposant ainsi à l'ouverture du clip et au glissement des tissus.

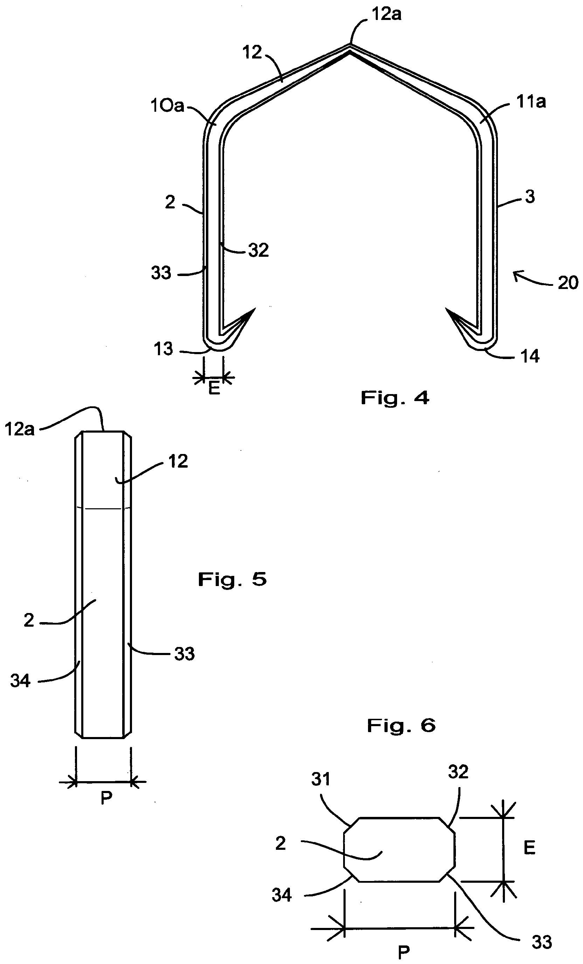

Naturellement, d'autres modes de réalisation sont possibles. Ainsi les figures 4, 5 et 6 représentent un autre mode de réalisation de la présente invention, dans lequel la section du clip est rectangulaire, de côtés E et P. Les tranches 31, 32, 33 et 34 du clip 20 sont biseautées, ce qui permet d'éviter l'endommagement des tissus par les tranches du clip. Pour la même raison, les sommets 10a et 11a sont arrondis. Par ailleurs, dans ce mode de réalisation, la base 12 est plus fine au niveau du sommet 12a afin de faciliter la déformation du clip.

## Revendications

1. Clip chirurgical d'anastomose vasculaire à déformation plastique ou à mémoire de forme, comportant une base de laquelle font saillie au moins deux branches (10, 11) présentant chacune au moins un crochet (15, 16) pointant vers la base (12), **caractérisé en ce que** la base forme un V présentant un sommet, le clip comportant trois zones de déformation privilégiée, constituées par le sommet et les zones de raccordement entre la base et les branches, de telle façon qu'après déformation, les branches restent sensiblement parallèles.

2. Clip chirurgical selon la revendication 1, **caractérisé en ce que** chaque branche forme avec son crochet un coin rentrant d'angle au sommet compris entre 30° et 80°.

3. Clip selon l'une quelconque des revendications précédentes, **caractérisé en ce que** la base (12) est déformable plastiquement.

4. Clip selon la revendication 3, **caractérisé en ce que** le clip est réalisé en titane.

5. Clip selon la revendication 1 ou la revendication 2, **caractérisé en ce que** le clip est réalisé en un alliage à mémoire de forme, la base (12) étant déformable par effet mémoire.

6. Clip selon l'une quelconque des revendications précédentes, **caractérisé en ce que** les branches (10, 11) présentent des extrémités libres arrondies.

7. Clip selon l'une quelconque des revendications précédentes, **caractérisé en ce que** les branches sont parallèles.

8. Clip chirurgical selon l'une quelconque des revendications précédentes, **caractérisé en ce que** le clip présente deux faces planes parallèles couvrant chacune la base et les deux branches, les deux faces planes étant situées à une distance relative P comprise entre 0,5 et 1,5 mm.

9. Clip selon la revendication 8, **caractérisé en ce que** le clip a une hauteur H, mesurée perpendiculairement aux faces planes parallèles, comprise entre 1 et 5 mm.

10. Clip selon l'une quelconque des revendications précédentes, **caractérisé en ce qu'**il est formé d'une pièce en un seul tenant.

## Patentansprüche

1. Plastisch verformbare oder formspeichernde chirurgische Klammer für eine Gefäßanastomose, welche eine Basis und mindestens zwei aus dieser hervorstehende Arme (10, 11) umfasst, wobei jeder mindestens einen zur Basis (12) zeigenden Haken (15, 16) aufweist, **dadurch gekennzeichnet, dass** die Basis v-förmig ist und einen Scheitel hat, wobei die Klammer drei bevorzugte Verformungsbereiche enthält, die durch den Scheitel und die Anschlussbereiche zwischen der Basis und den Armen gebildet werden, so dass die Arme nach der Verformung im Wesentlichen parallel zueinander bleiben.

**2.** Chirurgische Klammer nach Anspruch 1, **dadurch gekennzeichnet, dass** jeder Arm mit seinem Haken am Scheitel einen Innenwinkel von 30° bis 80° bildet.

**3.** Klammer nach einem der vorhergehenden Ansprüche, **dadurch gekennzeichnet, dass** die Basis (12) plastisch verformbar ist.

**4.** Klammer nach Anspruch 3, **dadurch gekennzeichnet, dass** die Klemme aus Titan besteht.

**5.** Klammer nach Anspruch 1 oder Anspruch 2, **dadurch gekennzeichnet, dass** die Klemme aus einer formspeichernden Legierung hergestellt wurde und die Basis (12) durch formspeichernde Wirkung verformbar ist.

**6.** Klammer nach einem der vorhergehenden Ansprüche, **dadurch gekennzeichnet, dass** die Arme (10, 11) an den freistehenden Enden abgerundet sind.

**7.** Klammer nach einem der vorhergehenden Ansprüche, **dadurch gekennzeichnet, dass** die Arme parallel zueinander stehen.

**8.** Chirurgische Klammer nach einem der vorhergehenden Ansprüche, **dadurch gekennzeichnet, dass** die Klemme zwei flache, parallele Seiten aufweist, die jeweils die Basis und die beiden Arme bedecken, wobei die beiden flachen Seiten in einem relativen Abstand P von 0,5 bis 1,5 mm voneinander entfernt sind.

**9.** Klammer nach Anspruch 8, **dadurch gekennzeichnet, dass** die Klammer eine Höhe H aufweist, die senkrecht zur den parallelen Seiten gemessen wird und zwischen 1 und 5 mm beträgt.

**10.** Klammer nach einem der vorhergehenden Ansprüche, **dadurch gekennzeichnet, dass** sie aus einem Stück geformt ist.

**Claims**

**1.** A surgical clip with plastic deformation or shape memory for vascular anastomosis, comprising a base from which at least two arms (10, 11) project, each having at least one hook (15, 16) pointing towards the base (12), **characterised in that** the base forms a V having a vertex, the clip comprising three favoured deformation zones, consisting of the vertex and the connection zones between the base and the arms, so that, after deformation, the arms remain substantially parallel.

**2.** A surgical clip according to claim 1, **characterised in that** each arm forms with its hook a corner with a re-entrant angle at the vertex of between 30° and 80°.

**3.** A clip according to any of the preceding claims, **characterised in that** the base (12) is plastically deformable.

**4.** A clip according to claim 3, **characterised in that** the clip is produced from titanium.

**5.** A surgical clip according to claim 1 or claim 2, **characterised in that** the clip is produced from a shape-memory alloy, the base (12) being deformable by memory effect.

**6.** A clip according to any of the preceding claims, **characterised in that** the arms (10, 11) have rounded free ends.

**7.** A clip according to any of the preceding claims, **characterised in that** the arms are parallel.

**8.** A surgical clip according to any of the preceding claims, **characterised in that** the clip has two parallel planar faces each covering the base and the two arms, the two planar faces being situated at a relative distance P of between 0.5 and 1.5 mm.

9. A clip according to claim 8, **characterised in that** the clip has a height H, measured perpendicular to the parallel planar faces, of between 1 and 5 mm.

10. A clip according to any of the preceding claims, **characterised in that** it is formed in a single piece.

**Fig. 1**

**Fig. 2**

**Fig. 3**

**Fig. 4**

**Fig. 5**

**Fig. 6**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 1712186 A
- FR 2557448
- FR 2588467
- EP 0656191 A
- EP 1462061 A
- US 2008173693 A
- WO 003099139 A
- FR 2899113